# EUROPEAN PATENT APPLICATION

(11) **EP 4 458 406 A1**
(43) Date of publication of application: **06.11.2024**
(21) Application number: 22921554.6
(22) Date of filing: 21.10.2022
(51) Int. Cl.: A61N 1/36, A61N 1/05, B81C 3/00

(54) **NEURAL INTERFACE SYSTEM**

(30) Priority: 21.01.2022 CN 202210074456
(71) Applicant: Wuhan Neuracom Technology Development Co., Ltd, Wuhan, Hubei 430000 (CN)
(72) Inventor: HUANG, Li, Wuhan, Hubei 430000 (CN); HUANG, Cheng, Wuhan, Hubei 430000 (CN); JI, Junwang, Wuhan, Hubei 430000 (CN); GAO, Jianfei, Wuhan, Hubei 430000 (CN); MA, Zhanfeng, Wuhan, Hubei 430000 (CN)
(74) Representative: De Tullio, Michele Elio
(86) International application number: PCT/CN2022/126567
(87) International publication number: WO 2023/138130

(57) **Abstract**

A neural interface system, comprising: at least one microneedle body (1), the microneedle body (1) comprising a liner and at least one body electrode (3), the liner having an arc-shaped structure, and the body electrode (3) being located on the outer side of the liner. When a retina of a human body is damaged, the microneedle body (1) is placed within an eye socket, and optic nerve cells are directly stimulated by using a signal capable of reconstructing vision, so that a visual perception function of a person is restored.

## Description

### TECHNICAL FIELD

The present invention belongs to the field of medical devices and specifically relates to a neural interface system.

### BACKGROUND ART

A neural interface is a communication system that does not rely on the normal output pathways consisting of peripheral nerves and muscles, which bypasses the peripheral nerves and muscle tissues and provides a novel pathway for exchanging information with external devices. The bidirectional communication between nerve cells and external devices can be achieved by stimulating nerve cells to generate action potentials through external devices, and recording the action potentials generated by nerve cells. Therefore, neural interfaces are widely used in the study and treatment of various neurological disorders, including the optic nerve system of human body.

Due to trauma, work environment factors, or aging, the retina in the human eyeball may be damaged or detached, leading to blindness or visual impairment. In recent years, as technology has continued to advance, artificial retina repair techniques have emerged. By directly converting optical signals into electrical signals, and then using the electrical signals to stimulate the cells in the inner layers of the retina, the vision of a patient can be restored to a certain extent. Currently, this technique has now become a research hotspot in the field of visual restoration. Implantable artificial retina electrodes are often made of rigid silicon electrodes or flexible non-silicon electrodes. The clarity of the image perceived by the patient depends on the number of electrodes implanted, which act like the pixels of a digital camera. The more electrodes there are, the clearer the perceived image will be. However, the conventional silicon/non-silicon technologies have fewer electrical contacts, making it impossible to differentiate the reading for optic nerve signals or differentiate the weak current stimulation for the optic nerve. Therefore, the conventional silicon/non-silicon technologies cannot meet the requirements of the human body for visual effects.

### SUMMARY

In response to the drawbacks and deficiencies of the prior art, an objective of the present invention is to provide a neural interface system, which is configured for directly stimulating nerve cells with signals transmitted from outside the eye when the human retina is damaged, so as to restore the visual perception function of a person.

The present invention adopts the following specific technical solution to realize the above objective. A neural interface system is provided, including at least one microprobe. The microprobe includes a liner and at least one microelectrode, wherein the liner is of an arc-shaped structure, and the microelectrode is located on the outer side of the liner.

In one of the optional embodiments, an in vitro device is further included, wherein the in vitro device includes an acquisition unit and a processing unit.

In one of the optional embodiments, the acquisition unit is configured to acquire an image, and the processing unit is configured to convert the image into a stimulus signal for reproducing the vision.

In one of the optional embodiments, the in vitro device further includes a first wireless coil, and the microprobe is provided with a second wireless coil.

The first wireless coil is configured to send the stimulus signal and the second wireless coil is configured to receive the stimulus signal.

In one of the optional embodiments, the microelectrode is implanted into an optic nerve.

In one of the optional embodiments, the neural interface system further includes a fixation member, wherein the microprobe is fixed within the eye socket by the fixation member.

In one of the optional embodiments, at least two microelectrodes have different length.

In one of the optional embodiments, a length of the microelectrode located in a middle region is greater than a length of the microelectrode located in two edge regions.

In one of the optional embodiments, a length of the microelectrode located in a middle region is smaller than a length of the microelectrode located in two edge regions.

In one of the optional embodiments, multiple microprobes are provided, and a microprobe array formed by the multiple microprobes is in a shape of cambered surface.

Compared with the prior art, the present invention has the following beneficial effects.

The present invention provides a neural interface system. When a retina of a human body is damaged, the microprobe is placed within an eye socket, and optic nerve cells are directly stimulated by using a signal capable of reconstructing vision, so that a visual perception function of a person is restored.

The present invention can customize a matched three-dimensional area array with multi-contact microprobes according to the degree of damage to the retina of the human eyeball, thus achieving multi-contact reading for individual neural action potentials and weak current stimulation for the optic nerve.

The present invention can achieve reading and writing of neural cell potential signals at the same location by designing independent microelectrodes and electrical contacts on a single microprobe, and enable the synchronous compensation and correction for visual information, thus providing a better visual experience for individuals with visual impairments.

### BRIEF DESCRIPTION OF DRAWINGS

To more clearly illustrate the technical solutions of the present invention, the following will briefly introduce the drawings to be used in the embodiments. It should be understood that the following drawings illustrate only certain embodiments of the present invention and therefore should not be viewed as limiting the scope of protection of the present invention. Similar numbering is used for similar components in the various drawings.
FIG. 1 is a structural schematic diagram of a neural interface system provided in the embodiments of the present invention;
FIG. 2 is a structural schematic diagram of a microelectrode provided in the embodiments of the present invention; and
FIG. 3 is a schematic diagram of circuit structure of an indium column connected to an integrated circuit chip provided in the embodiments of the present invention.

1- microprobe, 2- integrated circuit chip, 3- microelectrode, 4- wire, 5- electrical contact, 6- connecting wire, 7- indium column, 8- contact electrode, 9- transistor, 10- silicon substrate.

### DETAILED DESCRIPTION OF EMBODIMENTS

The technical solution in the embodiments of the present invention will be described clearly and completely, in conjunction with the drawings of the embodiments of the present invention. Obviously, the described embodiments are only part of the embodiments of the present invention, not all embodiments.

The components of embodiments of the present invention which are generally described and illustrated in the drawings herein can be arranged and designed in a variety of different configurations. Accordingly, the following detailed description of the embodiments of the present invention provided in the drawings is not intended to limit the scope of the present invention to be protected but merely represents selected embodiments of the present invention. Based on the embodiments in the present invention, all other embodiments obtained by those skilled in the art without making inventive efforts are within the scope of protection of the present invention.

In the following text, the terms "include", "provide", and their cognates, which may be used in various embodiments of the present invention, are only intended to indicate specific features, numbers, steps, operations, elements, components, or combinations thereof. They should not be interpreted as firstly excluding the presence of one or more other features, numbers, steps, operations, elements, components, or combinations thereof, or the possibility of adding one or more features, numbers, steps, operations, elements, components, or combinations thereof.

In addition, the terms "first", "second", and "third" are only used to distinguish the description and are not to be construed as indicating or implying relative importance.

Unless otherwise specified, all terms used herein (including technical and scientific terms) have the same meaning as that commonly understood by those ordinarily skilled in the field of various embodiments of the present invention. The terms (such as those defined in commonly used dictionaries) will be interpreted to have the same meaning as that of the context of the relevant technical field and will not be interpreted to have an idealized or overly formal meaning, unless clearly specified otherwise in various embodiments of the present invention.

### Embodiment 1

The present invention provides a neural interface system, including at least one microprobe. The microprobe includes a liner and at least one microelectrode, wherein the liner is of an arc-shaped structure, and the microelectrode is located on the outer side of the liner. Multiple microprobes are provided, and the microprobe array formed by the multiple microprobes is in a shape of cambered surface. The microelectrode is implanted into the optic nerve, which is configured for sending stimulus signals to the optic nerve or for acquiring optic nerve signals.

In one of the optional embodiments, an in vitro device is further included, wherein the in vitro device includes an acquisition unit and a processing unit. The acquisition unit is configured to acquire an image and the processing unit is configured to convert the image into a stimulus signal for reproducing the vision. The acquisition unit can be provided near the eye socket and the processing unit is provided near the ear. The acquisition unit can act as a human eye, which is configured for acquiring an image from the outside world. The processing unit converts the image into a stimulus signal that can reproduce vision, and sends the stimulus signal to the optic nerve via a microelectrode, so as to send the stimulus signal through the optic nerve to the nerve center of the brain to reconstruct the vision.

In practical application scenarios, the in vitro device further includes a first wireless coil, and the microprobe is provided with a second wireless coil. The first wireless coil is configured to send the stimulus signal and the second wireless coil is configured to receive the stimulus signal.

In one of the optional embodiments, the neural interface system further includes a fixation member, wherein the microprobe is fixed within the eye socket by the fixation member. The fixation member is a nail or adhesive, etc.

In practical application scenarios, at least two microelectrodes are of different length. In one of the optional embodiments, a length of the microelectrode located in a middle region is greater than a length of the microelectrode located in two edge regions. In another optional embodiment, a length of the microelectrode located in a middle region is smaller than a length of the microelectrode located in two edge regions.

In optional embodiments, the neural interface system includes multiple microprobes, with at least two microprobes having the liners with different curvature radii, thereby joining multiple microprobes into a spherical-shaped microprobe array. In the optional embodiments, the curvature radius of the liner located in the middle region is greater than the curvature radius of the liner located in the edge region.

In the embodiment, the neural interface system is configured to place the microprobe inside the eye socket when a retina of a human body is damaged and use signals capable of reconstructing vision to directly stimulate optic nerve cells, so as to enable the restoration of the visual perception function of a person.

### Embodiment 2

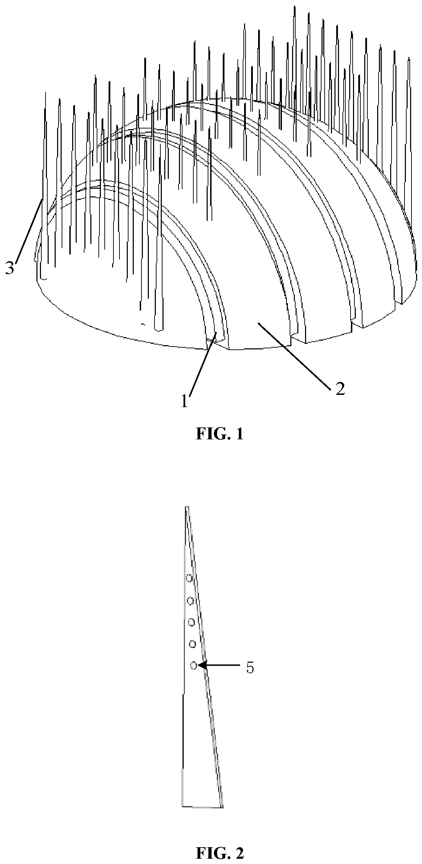

In conjunction with FIGS. 1 and 2, one embodiment of the present invention discloses a neural interface system. The neural interface system includes multiple microprobes l and an integrated circuit chip 2 (CMOS), wherein the multiple microprobes 1 are assembled together by means of a tethering device to form an array structure. The microprobe includes at least one microelectrode 3 and the microelectrode 3 is provided thereon with at least one electrical contact 5. Multiple microprobes can form a multi-contact area array, which enables the reading for the optic nerve signals or the weak current stimulation for the optic nerve through electrical contacts, thus improving spatial resolution and signal accuracy of the optic nerve. Meanwhile, bonding the microprobe l to the integrated circuit chip 2 can realize the function of signal input and signal output, thus effectively solving the problem of fewer contacts of the existing implantable artificial retinal microelectrode 3 using silicon/non-silicon technology.

According to one embodiment of the present invention, the tethering device is in the form of a hollow hemisphere, on which the microprobe l is fixed, so as to form a spike-shaped microprobe array. The tethering device is capable of fitting snugly with the eyeball when the microprobe array is inserted into the optic nerve. Therefore, the tethering device is not of a regular hollow hemisphere but is made of flexible material based on the bionic shape of the human eyeball. When implanted at the eye ground, there is no foreign sensation of friction due to frequent eye rotation.

### Embodiment 3

According to a specific embodiment of the present invention, the microprobe 1 includes multiple microelectrodes 3, wherein the microelectrodes 3 are independent of each other. The microelectrode 3 is provided thereon with multiple electrical contacts 5, wherein the individual electrical contacts 5 on the microelectrode 3 are independent of each other. Each electrical contact 5 can collect different and non-interfering electrical signals according to the degree of insertion into the neural tissue, or provide the weak current stimulation of different potentials at different cortical depths at the same location through integrated circuit chip 2, thereby forming a visual sensation with higher resolution. A microprobe 1 is provided with multiple microelectrodes 3, which can enable simultaneous reading operations of the signal and stimulation operations.

According to one embodiment of the present invention, the retina of the human eyeball is of a symmetrical structure, but the damage to the retina caused by disease or injury can vary in location. When a damage exists only on one side of the symmetric positions of the retina, the microprobes l corresponding to both sides can compensate for each other through reading and writing potential signals, thus restoring visual function. Specifically, the integrated circuit chip 2 can control the electrical contact 5 of the microprobe l on the undamaged side of the retina to read the potential signals of the optic nerve. Then, the integrated circuit chip 2 performs conversion of the imaging angle based on the specific position of the microprobe l inserted into the retina, and then controls the electrical contact 5 of the microprobe l on the symmetrical side to output corrected potential signals, thereby compensating and correcting the visual sensation of the human eyeball.

In another embodiment of the present invention, when the human retina is partially damaged, the functions of reading and stimulation can be realized separately with different microelectrodes 3 on the microprobe l at the same position. In other words, the integrated circuit chip 2 compares and corrects the electrical signals read and obtained by the human original retina relative to the potential signals converted from the image information captured by external devices. Then, the weak current stimulation to the optic nerve is performed through another microelectrode 3 on the microprobe 1 at the same location. With the development of 5G technology now, the signals mentioned above can be synchronized in real-time for compensation and correction, thus providing a better visual experience for individuals with visual impairments.

### Embodiment 4

According to an embodiment of the present invention, the material of the tethering device of the microprobe 1 is selected from a flexible transparent material. Due to varying degrees of damage to the human retina suitable for artificial retina, when the retina is completely or mostly detached due to trauma or disease, the original retina is no longer capable of normal imaging function. In this case, the artificial retina can fully replace the human retina. The obtained external image information is converted into electrical signals by the integrated circuit chip, and then the weak current stimulation is applied to the optic nerve and cell axons through the electrical contacts 5 of the microprobe l to generate visual sensation in the human body. At this point, the light rays and images outside the eyeball cannot be imaged on the human original retina. There are no specific requirements for the material and positioning of the tethering device and integrated circuit chip 2. Non-transparent materials can be applied, and the integrated circuit chip 2 can also be arranged at the front end of the retina or the tethering device. However, when the retina is partially damaged or partially detached and still retains some visual imaging function, arranging a hemispherical artificial retina on the inner surface of the eyeball retina will obstruct the entry of original light rays. In this case, the material of the tethering device for the microprobe l should be transparent. This means that while achieving reading or stimulating the optic nerve or cell axons with the electrical contacts 5, the partial sensory function of the human original retina will not be impeded.

According to an embodiment of the present invention, the integrated circuit chip 2 is also hollow and hemispherical in shape, which fits well with the tethering device of the neural interface system. The neural interface system is bonded together with the integrated circuit chip through the tethering device.

According to another embodiment of the present invention, the component formed by the neural interface system and the tethering device is electrically connected to the integrated circuit chip, wherein the integrated circuit chip is arranged outside the eyeball, thus reducing the impact of the heat generated by the integrated circuit chip on the imaging function of the human retina.

### Embodiment 5

In one embodiment of the present invention, the microprobes 1 in the neural interface system have the same length, and the tip points of multiple microprobes form a hemispherical cambered surface. According to another embodiment of the present invention, the microprobes 1 have different length, and are set differently according to the degree of retina damage, thus forming an unevenness multi-contact area array.

According to one embodiment of the present invention, the microprobes 1 in the neural interface system are evenly distributed on the tethering device. In another embodiment of the present invention, the microprobes l are unevenly distributed on the tethering device. The distribution density of the microprobes is set according to the degree of retina damage and sensory requirements. For individuals with damage at partial retinal, a denser array of microprobes can be arranged in the damaged region of the retina, and the regions without damage can be provided with sparser arrays of microprobes or provided with no array.

Specifically, the corresponding three-dimensional microprobe array can be arranged according to the examination results for the damage to the human retina, thus forming a multi-contact area array corresponding to the degree of the human retinal damage. The microelectrode 3 on the microprobe l can be trimmed, and a protective film is arranged at the cut edge of the trimmed microelectrode 3.

### Embodiment 6

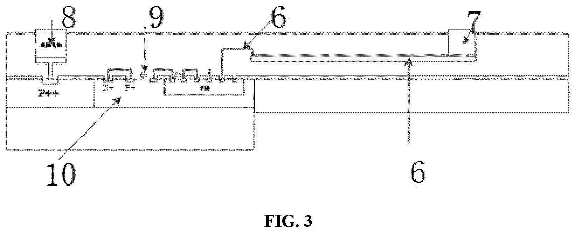

As shown in FIG. 3, the integrated circuit chip 2 is a readout circuit including a silicon substrate 10, wherein the silicon substrate is implanted thereon with a contact electrode 8 and a transistor 9. The silicon substrate is a P-type silicon substrate, and the indium column implanted on the integrated circuit chip 2 is electrically connected to the silicon substrate by means of a connecting wire 6.

The embodiments of the present invention also disclose a method of preparing a microprobe array including the following steps:
S1: using standard MEMS processing to fabricate the microprobe;
S2: processing at least one electrical contact on the microprobe;
S3: implanting the indium columns separately on the microprobe and the integrated circuit chip; and
S4: bonding the microprobe and the integrated circuit chip implanted with indium columns to form a microprobe assembly, and assembling multiple microprobe assemblies together through a tethering device to form a microprobe assembly array.

The multiple electrical contacts 5 on the microprobe 1 are connected to the indium columns 7 on the microprobe 1 via connecting wires 6. The connecting wires are metallic wires, and made of metal material. For example, the connecting wires are made of gold. In a practical application scenario, the electrical contact 5 is connected to a first soldering point on the microprobe via a connecting wire 6, and the indium column 7 is provided on the first soldering point, thereby connecting the electrical contact 5 to the integrated circuit chip 2.

Furthermore, the electrical contacts 5 on the microelectrodes 3 can be distributed in the same column or in different columns, which can depend on the width of the microelectrodes 3 and the actual situation. When the electrical contacts 5 are distributed in different columns, the electrical contacts 5 of neighboring columns can be arranged in a staggered manner.

According to an embodiment of the present invention, the electrical contact 5 is configured to acquire optic nerve signals and transmit the acquired optic nerve signals to the integrated circuit chip 2. The integrated circuit chip 2 is configured, on the one hand, to receive the optic nerve signals acquired by some of the electrical contacts 5, and on the other hand, to transmit the electrical signals to some of the electrical contacts 5, thus restoring the visual perception function of a person. That is to say, after bonding the microprobe 1 to the integrated circuit chip 2, the functions of signal input and signal output can be realized, the action potentials of individual nerve cell can be read, and the weak current stimulation can be realized.

The above-mentioned embodiments only express several implementation modes of the present invention, and the descriptions thereof are relatively specific and detailed, but should not be construed as limiting the scope of the present invention. It should be pointed out that those ordinarily skilled in the art may make several modifications and improvements without departing from the concept of the present invention, and these all belong to the protection scope of the present invention. Therefore, the protection scope of the present invention should be based on the appended claims.

## Claims

1. A neural interface system, comprising at least one microprobe, **characterized in that** the at least one microprobe comprises a liner and at least one microelectrode; the liner is of an arc-shaped structure; and the at least one microelectrode is located on an outer side of the liner.

2. The neural interface system according to claim 1, further comprising an in vitro device, wherein the in vitro device comprises an acquisition unit and a processing unit.

3. The neural interface system according to claim 2, wherein the acquisition unit is configured to acquire an image and the processing unit is configured to convert the image into a stimulus signal for reproducing a vision.

4. The neural interface system according to claim 2, wherein the in vitro device further comprises a first wireless coil, and the at least one microprobe is provided with a second wireless coil; and
the first wireless coil is configured to send a stimulus signal and the second wireless coil is configured to receive a stimulus signal.

5. The neural interface system according to claim 1, wherein the at least one microelectrode is implanted into an optic nerve.

6. The neural interface system according to claim 5, wherein the neural interface system further comprises a fixation member, and the at least one microprobe is fixed within an eye socket by the fixation member.

7. The neural interface system according to claim 1, wherein at least two microelectrodes of the at least one microelectrode have different length.

8. The neural interface system according to claim 7, wherein a length of a microelectrode located in a middle region is greater than a length of a microelectrode located in two edge regions.

9. The neural interface system according to claim 7, wherein a length of a microelectrode located in a middle region is smaller than a length of a microelectrode located in two edge regions.

10. The neural interface system according to any one of claims 1 to 9, wherein multiple microprobes are provided, and a microprobe array formed by the multiple microprobes is in a shape of cambered surface.
